# EUROPEAN PATENT SPECIFICATION

(11) **EP 2 731 743 B1**
(45) Date of publication and mention of the grant of the patent: **13.04.2016**
(21) Application number: 11764856.8
(22) Date of filing: 12.07.2011
(51) Int. Cl.: B22F 9/04, B22F 1/00, C22C 5/02, A61K 33/24, A61K 36/752, A61K 9/127, A61K 36/23, A61K 36/48, B82Y 30/00

(54) **BIOSYNTHESIS PROCESS FOR PREPARATION OF NANO GOLD**
BIOSYNTHESE-VERFAHREN ZUR HERSTELLUNG VON NANOGOLD
PROCÉDÉ DE BIOSYNTHÈSE POUR LA PRÉPARATION DE NANO-OR

(43) Date of publication of application: 21.05.2014
(73) Proprietor: Bendale, Yogesh Narayan, Pune 411052 (IN); Bendale, Vineeta Yogesh, Pune 411052 (IN)
(72) Inventor: Bendale, Yogesh Narayan, Pune 411052 (IN); Bendale, Vineeta Yogesh, Pune 411052 (IN)
(74) Representative: Patel, Binesh
(86) International application number: PCT/IB2011/053097
(87) International publication number: WO 2013/008061

(56) References cited:
- WO-A1-2005/095031
- WO-A1-2010/046903
- WO-A1-2010/087869
- WO-A2-2007/031888
- US-A1- 2004 091 552
- TAI Y ET AL: "One-step synthesis of highly biocompatible multi-shaped gold nanostructures with fruit extract", THE INSTITUTION OF ENGINEERING AND TECHNOLOGY. JOURNAL,, vol. 5, no. 2, 1 June 2011 (2011-06-01), pages 52-59, XP006037685, ISSN: 1751-875X, DOI: 10.1049/IET-NBT:20100028
- ROY N ET AL: "A detailed study on the antioxidant activity of the stem bark of Dalbergia sissoo Roxb., an Indian medicinal plant", FOOD CHEMISTRY, ELSEVIER LTD, NL, vol. 126, no. 3, 1 June 2011 (2011-06-01), pages 1115-1121, XP027601017, ISSN: 0308-8146, DOI: 10.1016/J.FOODCHEM.2010.11.143 [retrieved on 2011-01-13]
- ROCKTOTPAL KONWARH ET AL: "Biomimetic preparation of polymer-supported free radical scavenging, cytocompatible and antimicrobial green silver nanoparticles using aqueous extract ofpeel", COLLOIDS AND SURFACES. B, BIOINTERFACES, ELSEVIER, AMSTERDAM, NL, vol. 84, no. 2, 16 January 2011 (2011-01-16), pages 338-345, XP028167297, ISSN: 0927-7765, DOI: 10.1016/J.COLSURFB.2011.01.024 [retrieved on 2011-01-25]
- PRATHNA T C ET AL: "Biomimetic synthesis of silver nanoparticles by Citrus limon (lemon) aqueous extract and theoretical prediction of particle size", COLLOIDS AND SURFACES. B, BIOINTERFACES, ELSEVIER, AMSTERDAM, NL, vol. 82, no. 1, 1 January 2011 (2011-01-01), pages 152-159, XP027404155, ISSN: 0927-7765 [retrieved on 2010-10-07]
- NILESH C. SHARMA ET AL: "Synthesis of Plant-Mediated Gold Nanoparticles and Catalytic Role of Biomatrix-Embedded Nanomaterials", ENVIRONMENTAL SCIENCE & TECHNOLOGY, vol. 41, no. 14, 1 July 2007 (2007-07-01), pages 5137-5142, XP055071128, ISSN: 0013-936X, DOI: 10.1021/es062929a
- J. L. GARDEA-TORRESDEY ET AL: "Formation and Growth of Au Nanoparticles inside Live Alfalfa Plants", NANO LETTERS, vol. 2, no. 4, 1 April 2002 (2002-04-01), pages 397-401, XP055071126, ISSN: 1530-6984, DOI: 10.1021/nl015673+

## Description

### Field of Invention

The present invention is an environment friendly process to obtain nano gold. The present invention also finds applicability in the field of nanotechnology, biotechnology, green technology and material science.

### Object of Invention

The object of the invention is production of thermally stable and less toxic nano gold of particle size ranging from 10nm-1000nm through bio synthesis. The present invention is a merger between biotechnology and green technology for production of nano gold without toxic waste. The nano gold also finds application in various fields including but not limited to technology considering its stability, robust nature, particle size and low toxicity. Further object of the present invention is to establish an alternative environment friendly process for the production of nano gold without toxic waste.

### Background Art

WO 2007/031888 A2 describes the purification of Pt metal by heating it to 600 °C until it is red hot followed by boiling it in a solution comprising Dolichos Biflorous. Subsequently, the Pt metal is granulated to small particles. This process results in purified metal nanoparticles.

Further, the production of silver nanoparticles has been reported (Rocktotpal Konwarh et al., Biometric preparation of polymer-supported free radical scavenging, cytocompatible and antimicrobial green silver nanoparticles using aqueous extract of peel. Colloids and Surfaces. B, Biointerfaces, Elsevier, vol. 84, no. 2, 16 January 2011, pages 338-345). Said silver nanoparticles are obtained by applying a process of reducing AgNO₃ with citrus sinensis peel or orange peel resulting in Ag nanoparticles with reduced particle size. A similar process the production of silver nanoparticles, however, by applying juice of lemon or with citric acid is known from a report published by T. C. Prathna et al., Biomimetic synthesis of silver nanoparticles by Citrus limon (lemon) aqueous extract and theoretical prediction of particle size, Colloids and Surfaces. B, Biointerfaces, Elsevier, vol. 82, no. 1, 1 January 2011, pages 152-159.

Therapeutic applicability of gold is known since ancient times. Orally administrable Gold based products are known in traditional and alternative medicine. Further, Gold based products obtained through chemical reduction process, ingestible and injectable, are also available for treatment of humans and animals. Gold salts obtained through chemical reduction are noticed to cause side effects due to toxicity thereby limiting its therapeutic application. Despite chemical purification, impurities are found on the surface of the gold particles, which causes various side effects upon administration in humans and animals. The toxicity of the gold based product causes side effects like nausea, vomiting, diarrhoea, dermatitis, haematological disorder. The main reason attributed to the toxicity is the change in oxidation state after administration and the carrier molecule. The orally administrable gold product obtained through chemical reduction, for instance Auranofin or Ridaura, are noted to be less therapeutically effective than injectable gold salts.

With the advent of nano gold particles obtained through chemical reduction, under water arcing, electrochemical process etc. the therapeutic applicability of gold has improved. However, the colloidal gold, obtained through the chemical reduction process results in stable and partially stable gold colloids or gold nano suspensions and are found to be less effective when orally administered. Product nano gold obtained through electrochemical process, which is patent pending, is described to have cleaner surface thereby increasing its efficacy in vivo and the particle size range from 8nm to 100nm (PCT/US2010/041427).

Gold based products known for its anti inflammatory properties and bacteriostatic effect are used in the treatment of rheumatoid arthritis, tuberculosis, cancer, asthma, HIV, Malaria etc. However, the toxicity and in vivo oxidation factor has caused its restricted use in treatment of animals and humans.

Further, gold nano particles, more robust and stable, are one of the most preferred nano materials in technological applications especially biomedical applications such as bio imaging in the present scenario.

The state of art indicates existence of production of nano gold through various methods.

US 2004/0091552 A1 refers to ultra-fine gold particles produced by a process including the steps: (i) supply a gold rod from a feeder or supply a gold foil into a tank filled with pressurized water, (ii) evaporate gold by igniting a gas mixture in a combustion chamber placed inside the tank; thereby, ultra-fine gold particles are dispersed in water. By this process nanometer-sized particles are obtained for which a therapeutic use is suggested.

WO 2010/087869 A1 shows chemically pure metal nano-particle colloids, the metal being for example gold. The metal nano-particle colloids are used inter alia as catalysts, chemical and biological sensors, medical products such as antibacterial agents and water purifying agents. They are produced by putting a gold target in a container filled with water and focusing a laser beam on the target to produce the nano-particles.

According to WO 2010/046903 A1 gold nanoparticles are provided produced by preparing a solution comprising auric chloride, which is reduced by trisodium citrate. The gold nanoparticles are used to perpetuate the stemness of stem cells.

In addition, gold nano-particles have been reported, which are biocompatible for use in therapy of animals or humans (Y. Tai et al., One-step synthesis of highly biocompatible multi-shaped gold nanostructures with fruit extract. The institution of engineering and technology. Nanobiotechnology. Volume 5, Issue 2, June 2011, p. 52 - 59.). The gold nanoparticles are produced by a process comprising the addition of an aqueous HAuCl₄ solution in drops to a flask containing an extract of fruits followed by heating, stirring and cooling. Orange (Citrus x sinensis), papaya (carica papaya), peach (prunus persica) and lemon (citrus x lemon) fruits were used.

According to WO 2005/095031 A1 the production of metal nanoparticles, e.g. noble metals such as Au, is presented. Said nanoparticles are obtained by applying a process of reducing the corresponding salts while using plant material extracts such as Cicer arietinum resulting in purified metal nanoparticles.

According to a report properties of the bark of Dalbergia sissoo such as aqueous extracts or methanol extracts in several applications have been analyzed (N. Roy et al., A detailed study on the antioxidant activity of the stem bark of Dalbergia sissoo Roxb., an Indian medicinal plant. Food Chemistry, Elsevier, vol. 126, no. 3, 1 June 2011, pages 1115-1121.). The antioxidant activity of said extracts is used for producing Au nanoparticles from HAuCl₄.

According to a study, a preparation of a solution comprising an Au-salt (potassium tetrachloroaurate) and seeds of the plant material Sesbania drummondii is suggested (Nilesh C. Sharma et al., Synthesis of Plant-mediated Gold Nanoparticles and Catalytic role of Biomatrix-embedded Nanomaterials. Environmental Science & Technology, vol. 41, no. 14, 1 July 2007, pages 5137-5142.). From this solution gold nanoparticles are formed, which are taken up by the plant material. Subsequently, the plant material is cryo-ground in liquid nitrogen, whereby the gold nanoparticles are set free.

According to a further report, a preparation of a solution comprising an Au-salt (potassium tetrachloroaurate) and seeds of the plant material Alfalfa is suggested (J. L. Gardea-Torresdey et al., Formation and Growth of Au Nanoparticles inside Live Alfalfa Plants. Nano Letters. vol. 2, no. 4, 1 April 2002, pages 397-401). From this solution reduced gold nanoparticles are formed, which are taken up by the plant material. Said process results in purified metal nanoparticles.

The process of production of 'swarna bhasma' is known to obtain nano gold through bio synthesis. The swarna bhasma, comprising of gold containing globular particles having an average size of 56nm-57nm, has been an integral part of traditional medicine in India. The traditional method comprises of (1) purification with the aid of sesame oil (Sesamum Indicum Linn.), butter milk, cow's urine, sour gruel processed from Oryza Sativa, and extract of Dolichos biflorous Linn (2) special purification with the aid of hematite and rock salt and heating process (3) process of incineration by addition of mercury and sulphur in required proportion. The resultant product is brownish red powder 'swarna bhasma' (BrownCL et al, Gold Bulletin 2007).

The production and use of 'swarna bhasma' is further discussed in PCT/ IN2008/000816, where the 'swarna bhasma' produced through traditional method is claimed in a method to perpetuate stemness in stem cell therapy. However, the process or the product discussed in the said application is not similar to the present invention.

An alternative process of producing gold nanoparticles should be provided by present invention.

### Summary of Invention

The present invention encompasses a process to obtain nano gold according to claim 1. Specifically, the novel process is carried out with the aid of plant materials and in different phases.

Phase I of the process is that of purification of gold metal with the aid of Dalbergia Sissoo and Dolichos Biflorous. Phase II involves particle size reduction of the purified gold with the aid of Citrus Acida and Ferula. Phase III is of incineration for production of purified nano gold of size ranging from 10nm-1000nm.

The present invention is novel bio synthesis different from any of the existent art including the process involved in production of 'swarna bhasma'. The plant materials used are different and the special purification process emphasised in the production of swarna bhasma with the aid of Mercury and Sulphur is not a part of this invention. Further, the temperature and the process of incineration differ in the present invention. The present invention uses Dolichos Biflorus independently of any other plant or organic material in the purification phase after the metallic gold is boiled with Dalbergia Sissoo. The traditional method involves the use of Dolichos Biflorous in conjuction with Cow's Urine, buttermilk etc.

The product obtained through said process is pinkish brown or yellow in colour depending on the desired particle size. The product is thermally stable and also non-toxic when administered in specific dosage.

### Detailed Description

The present invention relates to a process to obtain nano gold according to claim 1, in particular to the bio synthesis of nano gold particles through different steps of purification, trituration and incineration with the use of plant materials. The present invention deals with the process of obtaining nano gold through bio synthesis that is carried out in three phases i.e, the first phase is of bio purification of the metallic gold foils with the aid of extract of Dalbergia Sissoo and extract of Dolichos biflorous and the second phase is of trituration with other plant materials, such as Citrus and Ferula, for particle size reduction and third phase is of heating resulting in novel nano gold devoid of toxicity when administered in specific dosage, orally administrable, with higher adsorption capability. The essence of the present invention also include the use of Dalbergia Sissoo in purification process of the metal and use of plant materials Citrus and Ferula, in the process of particle size reduction, which is not known in the prior art.

The present inventive process produces nano gold of size 10nm - 1000nm through bio synthesis. The present invention not only is a novel bio process to produce nano gold particle but also includes the novel use of plant materials in metal purification and particle size reduction of metals. The use of plant materials taxonomically classified under Genus 'Dalbergia' or in specific Dalbergia Sissoo or any other neoflavonoids having characteristics of the said compound in the purification process of the gold metal before particle size reduction is not known. The present invention provides for the use of mixture, in equal proportion by weight, of Citrus Acida, in liquid form, and Ferula in dry form, in particle size reduction of the gold metal purified through bio synthesis into nano gold of size 10nm-1000nm. Gold purified through any other method bringing same quality of the product of phase I is envisaged in use for particle size reduction with the aid of Citrus Acida and Ferula. Use of any plant material under taxonomical Genus 'Citrus' and 'Ferula' are anticipated in the present invention. Further, use of plant materials or chemical complexes with similar properties is anticipated in the said process of purification and particle size reduction of metals.

Dolichos biflorous is commonly used as an astringent, diuretic and tonic. The enzymes found in Dolichos biflorous have been identified as Urease, Allantoinase, Ribonuclease, Nicotinamide deaminase, β-n-acetylglucosaminidase, α- and β-galactosidase, α-mannosidase and β-glucosidase. In the present invention it is used during the phase of purification after the metallic gold is boiled with Dalbergia Sissoo, which is a novel utility.

Citrus Acida, taxonomical classification Genus 'Citrus' is commonly found in Asia. The juice of Citrus Acida contains Citric Acid, Mallic Acid, Phosphoric Acid and Salt. Medicinal properties of Citrus Acida are well known and it is also used as ingredient in food. The use of Citrus Acida in particle size reduction of metal is not known in the state of art. The present invention utilizes juice of Citrus Acida for particle size reduction of gold metal through bio synthesis. Plant under Genus 'Citrus' such as Limonum, Medica, Aurantium, Garcinia Pedunculata or any other plant material containing citric acid and other contents found in Citrus Acida or compounds or chemical complexes that bring similar effect is anticipated in the present invention.

Plant materials under the Genus Ferula are known for their medicinal properties and are also used as food ingredient. Ferula Narthex, Ferula Asafoetida and Ferula Foetida are generally native to Iran and Afghanistan and has been widely used since ancient times in traditional medicine. However, the combination of Ferula with juice of Citrus Acida for particle size reduction of metal is not known in the state of art or traditional medicine. The present invention establishes a unique and non obvious use of plant materials under taxonomical Genus 'Ferula' and 'Citrus'.

One embodiment of present invention is the use of Ferula Narthex in conjunction with Citrus Acida in adequate proportion to reduce particle size of purified gold metal.

Phase I of the process involves bio purification of Gold metal, which is the first step in the process for production of nano gold. Gold metal, in the form of foils or otherwise, is heated, preferably at about 600°c, until red hot. The temperature of 600°c cannot be considered as limiting. Red hot gold metal is boiled in oil extract of Dalbergia Sissoo for several hours preferably minimum of seven (7) hours. The minimum time of seven (7) hours required for boiling cannot be considered limiting. The resultant product obtained after boiling with Dalbergia Sissoo is then boiled in extract of Dolichos biflorus for several hours, minimum of seven (7) hours but not limiting, to obtain metallic powder. The resultant product, i.e., metallic powder is put through the process of boiling with Dalbergia Sissoo and consequent boiling in extract of Dolichos biflorous several times in order to obtain organically purified gold through biosynthesis. The process is required to be repeated ten (10) times but the repetition of the process to the extent of ten times cannot be considered limiting being a natural process.

Phase II of novel process involves trituration. The product of Phase I is triturated with equal portion, by weight, of mixture of juice of Citrus Acida and Ferula Narthex in dry form, at temperature ranging from 22°c to 45°c until dry. The dry product obtained is further put through the process of trituration with mixture of Citrus Acida and Ferula Narthex repeatedly, minimum of ten (10) times, to reach the optimum finely divided desired particle size. The proportion of juice of Citrus Acida and dry Ferula in the mixture has to be equal by weight. The repetition of the process (10) times should not be considered as limiting. The desired particle size is the deciding factor of the number of repetition.

Phase III of the present inventive process involves incineration. The dry product after attaining its optimum particle size is exposed to high temperature ranging from 300°c to 950°c and cooled gradually until dry. The minimum temperature of exposure is preferably 300°c. This process of heating and cooling is repeated several times, minimum 10 times, until the dry product nano gold is obtained. The colour of the product varies from mild pinkish brown to yellow depending on the desired particle size. The number of times the process is repeated to obtain the product of desired particle size should not be considered as limiting being a natural process. Biomass, generally cow dung is used as fuel for the purpose of heating so as to ensure that the optimum range of heating and cooling is maintained. Use of biomass ensures that maximum optimal temperature is obtained prior to gradual cooling until dry product is obtained. This process of incineration is carried out in apparatus that provides uniform heating.

The pinkish brown or yellowish coloured fine dust of nano gold is biologically pure and particle size range from 10nm to 1000nm.

## Claims

1. A process to obtain nano gold comprising the following steps:
(a) subjecting gold metal to heat until red hot,
(b) boiling of red hot gold with extract of Dalbergia Sissoo,
(c) boiling of the gold subjected to step (b) in extract of Dolichos Biflorous,
(d) trituration of the product obtained from step (c) with mixture of Citrus Acida and Ferula Narthex until dry,
(e) Incineration of the product obtained through step (d), by a method that subjects the product of step (d) to gradual increase in temperature until the optimum maximum temperature is obtained,
(f) Gradual cooling of product of step (e) until dry.

2. The use of Dalbergia Sissoo plant material or its extracts in step b) of the process according to claim 1 for gold metal purification.

3. The use of Dolichos Biflorous plant material or its extracts in step c) of the process according to claim 1 for gold metal purification.

4. The use of Citrus Acida or Ferula Narthex plant material or its extracts in step d) of the process according to claim 1 for particle size reduction of the purified gold.

## Patentansprüche

1. Ein Prozess zur Erlangung von Nanogold, der die folgenden Schritte aufweist:
(a) Erhitzen von Goldmetall bis zum Rotglühen,
(b) Sieden des rotglühenden Goldes mit Extrakt aus Dalbergia Sissoo,
(c) Sieden des Goldes, das Schritt (b) unterworfen war, in Extrakt aus Dolichos Biflorous,
(d) Zerreiben des aus Schritt (c) erlangten Produktes mit einem Gemisch aus Citrus Acida und Ferula Narthex bis es getrocknet ist,
(e) Veraschung des aus Schritt (d) erlangten Produktes, durch ein Verfahren, das das Produkt aus Schritt (d) einer schrittweisen Temperaturerhöhung aussetzt, bis die optimale Höchsttemperatur erreicht ist,
(f) Schrittweise Abkühlung des Produktes aus Schritt (e) bis es getrocknet ist.

2. Die Verwendung des Dalbergia Sissoo-Pflanzenmaterials oder die Extrakte aus Schritt b) des Prozesses gemäß Anspruch 1 für die Goldmetall-Reinigung.

3. Die Verwendung des Dolichos Biflorous-Pflanzenmaterials oder die Extrakte aus Schritt c) des Prozesses gemäß Anspruch 1 für die Goldmetall-Reinigung.

4. Die Verwendung des Citrus Acida- oder Ferula Narthex-Pflanzenmaterials oder die Extrakte aus Schritt d) des Prozesses gemäß Anspruch 1 für die Teilchengrößenreduktion des gereinigten Goldes.

## Revendications

1. Processus pour obtenir du nano or comprenant les étapes suivantes :
(a) soumission de l'or métallique à la chaleur jusqu'au rouge chaud,
(b) ébullition de l'or rouge chaud avec un extrait de Dalbergia sissoo,
(c) ébullition de l'or soumis à l'étape (b) dans un extrait de Dolichos biflorus,
(d) trituration du produit obtenu à partir de l'étape (c) avec un mélange de Citrus acida et de Ferula Narthex jusqu'à ce qu'il soit sec,
(e) incinération du produit obtenu à l'étape (d), par un procédé qui soumet le produit de l'étape (d) à une augmentation graduelle de la température jusqu'à ce que la température maximale optimale soit obtenue,
(f) Refroidissement graduel de produit de l'étape (e) jusqu'à ce qu'il soit sec.

2. Utilisation de matériel végétal de Dalbergia sissoo ou de ses extraits à l'étape b) du procédé selon la revendication 1 destinée à la purification de l'or métallique.

3. L'utilisation de matériel végétal de Dolichos Biflorus ou de ses extraits dans l'étape c) du procédé selon la revendication 1 destinée à la purification de l'or métallique.

4. Utilisation de matériel végétal de Citrus acida ou de Ferula Narthex ou de ses extraits à l'étape d) du procédé selon la revendication 1 destinée à la réduction de la taille des particules de l'or purifié.
